# EUROPEAN PATENT APPLICATION

(11) **EP 3 023 401 A1**
(43) Date of publication of application: **25.05.2016**
(21) Application number: 14194053.6
(22) Date of filing: 20.11.2014
(51) Int. Cl.: C07C 1/22, C07C 15/085, B01J 23/72

(54) **Improvements relating to hydrogenolysis of phenyl alcohols**

(71) Applicant: Shell Internationale Research Maatschappij B.V., 2596 HR Den Haag (NL)
(72) Inventor: BASAK, Kaushik, Bengaluru 560048 (IN); BOELENS, Minne, 1031 HW Amsterdam (NL); FRIEDEL, Frank, 06116 Halle (DE); KLEMT, Andreas, 04129 Leipzig (DE); VAN MOURIK, Arian, 1031 HW Amsterdam (NL)
(74) Representative: Matthezing, Robert Maarten

(57) **Abstract**

A method for the hydrogenolysis of a phenyl alcohol to eliminate a hydroxyl group therefrom, the method comprising contacting a feed comprising the phenyl alcohol and a hydrogenating agent with a heterogeneous catalyst comprising copper and silica at a reaction temperature of less than 140 °C, wherein the catalyst comprises copper (calculated as CuO) in an amount in the range of from 71 to 90 % by weight of the catalyst. A process for the preparation of an alkylene oxide is also disclosed.

## Description

### Field of the Invention

This invention relates the hydrogenolysis of phenyl alcohols. In particular, though not exclusively, the invention relates to the hydrogenolysis of phenyl alcohols formed during the production of alkylene oxides, and to processes for producing alkylene oxides involving hydrogenolysis of phenyl alcohols.

### Background to the Invention

Propylene oxide (PO) is an essential building block for a variety of chemicals and products. Global production of PO exceeds seven million tonnes per annum.

Direct oxidation of propylene with air or oxygen to form PO tends to provide low yields. PO is therefore most commonly produced with the help of a chemical mediator.

One known process comprises contacting a phenyl hydroperoxide, in particular an alkyl phenyl hydroperoxide, and propylene with a heterogeneous epoxidation catalyst and withdrawing a product stream comprising PO and a phenyl alcohol.

One phenyl hydroperoxide that can be used in this epoxidation process is ethylbenzene hydroperoxide (EBHP), in which case the alcohol obtained is 1-phenylethanol. The 1-phenylethanol is typically converted into styrene by dehydration.

Another phenyl hydroperoxide that can be used in such epoxidation is cumene hydroperoxide (2-hydroperoxypropan-2-ylbenzene), typically obtained by reacting cumene ((1-methylethyl)benzene)) with oxygen or air. Cumene hydroperoxide is reacted with propylene in the presence of an epoxidation catalyst to yield PO and cumyl alcohol (2-phenylpropan-2-ol). Cumyl alcohol, also referred to as dimethylphenylcarbinol (DMPC), is typically converted into cumene with the help of a heterogeneous catalyst and hydrogen, by hydrogenolysis. The cumene may then be re-used in the process.

The hydrogenolysis of phenyl alcohols, in particular the conversion of DMPC to cumene, is discussed in numerous documents such as WO 2005/030744 A1, WO 2005/030741 A1, WO 03/024900 A1, WO 2009/110485 A1 and WO 2005/030684 A1 in the name of Sumitomo.

EP 1433770 A1 describes a process for producing cumene by subjecting cumyl alcohol to hydrogenolysis in the presence of a copper-based heterogeneous catalyst. A wide range of copper catalysts are described in EP 1433770 A1, including copper, Raney copper, copper-chromium, copper-zinc, copper-chromium-zinc, copper-silica and copper-alumina.

Similarly, EP 1437350 A1 discloses a process for the preparation of propylene oxide, which process comprises a hydrogenolysis step comprising subjecting cumyl alcohol to hydrogenolysis in the presence of a heterogeneous catalyst.

A wide range of catalysts may be used in the hydrogenolysis step of EP 1437350 A1 such as cobalt, nickel, palladium, copper and zinc metal catalysts. Copper catalysts are as indicated as being preferred in EP 1437350 A1.

The preferred hydrogenolysis catalyst exemplified in both EP 1433770 A1 and EP 1437350 A1 is a copper-chromium catalyst. However, this catalyst is shown therein to require high temperatures of 220 °C and 180 °C, respectively, in order to achieve high conversion levels.

US 7319177 B2 is also concerned with the conversion of cumyl alcohol to cumene. Accordingly, said document describes a two-step process comprising the dehydration of cumyl alcohol to α-methylstyrene in the presence of a dehydration catalyst, followed by the hydrogenation of said α-methylstyrene to cumene in the presence of a hydrogenation catalyst.

The dehydration catalyst in US 7319177 B2 may be selected from acids such as sulphuric acid, phosphoric acid and p-toluenesulfonic acid and metal oxides such as activated alumina, titania, zirconia, silica-alumina and zeolites, whilst the hydrogenation catalyst may be selected from a solid catalyst containing a metal of Group 10 or 11 of the Periodic Table of the Elements such as nickel, palladium, platinum and copper. Palladium and copper are indicated to be preferred and various copper-based and palladium-based catalysts are listed.

Example 1 of US 7319177 B2 describes the conversion of cumyl alcohol to cumene in the presence of activated alumina as the dehydration catalyst and 60 wt. % copper-silica as the hydrogenation catalyst. It is apparent that the hydrogenation step took place at a temperature of 205 °C.

WO 2005/005350 A2 describes a process for producing alkylbenzene from an alkylphenyl alcohol in the presence of a catalyst comprising a Group VIII or a Group IB metal.

Various commercial catalysts are described and tested in WO 2005/005350 A2, including a copper-silica catalyst available under the trade designation "T-366" catalyst from Süd Chemie (comprising 50-70 wt. % CuO) and a copper chromite catalyst available from Süd Chemie under the trade designation "G-22/2". In addition, a Cu/Zn/Zr catalyst, prepared according to Example 3 of US 5475159 A, is also tested.

Example IB of WO 2005/005350 A2 details the conversion of cumyl alcohol in the presence of reduced "T-366" catalyst wherein the conversion takes place at reflux, i.e. at temperatures of approximately 201 °C. Whilst the conversion of cumyl alcohol is approximately 100%, there is no information in said example regarding the amount of by-products.

Example IIC of WO 2005/005350 A2 describes the conversion of cumyl alcohol in the presence of reduced "T-366" catalyst at a temperature of 150 °C, wherein after 200 hours of operation, the dried product contained 9.7 wt. % 2-phenyl-2-propanol, 88.4 wt. % of cumene, 0.1 wt. % alpha-methyl styrene, 0.1 wt. % of i-propylcyclohexane and 1.7 wt. % of cumene dimer. That is to say, the conversion of cumyl alcohol was 62.0 % and the amount of i-propylcyclohexane and cumene dimer by-products totalled 1.8 wt. %.

In contrast, it is indicated in Example IIC that when the reaction was operated at 180 °C, then the conversion was increased and the product contained less than 5 wt. % of 2-phenyl-2-propanol. However, the amount of by-products is not specified.

It is an object of the invention to provide a process for the hydrogenolysis of a phenyl alcohol, e.g. as part of an alkylene oxide production process, which can not only achieve high conversion levels of phenyl alcohol to the corresponding alkyl phenol but which also leads to reduced amounts of by-products resulting from ring hydrogenation, condensation reactions and cracking.

### Statements of the Invention

In the present invention, it has been surprisingly found that the use of a specific heterogeneous hydrogenolysis catalyst at lower temperatures, not only leads to high conversion of phenyl alcohol, but also minimises the amount of unwanted by-products.

Accordingly, in a first aspect of the present invention there is provided a method for the hydrogenolysis of a phenyl alcohol to eliminate a hydroxyl group therefrom, the method comprising contacting a feed comprising the phenyl alcohol and a hydrogenating agent with a heterogeneous catalyst comprising copper and silica at a reaction temperature of less than 140 °C, wherein the catalyst comprises copper (calculated as CuO) in an amount in the range of from 71 to 90 % by weight of the catalyst.

The method of the present invention has significant benefits in the context of industrial hydrogenolysis, e.g. in propylene oxide production. Firstly, lower operating temperatures offer the opportunity for energy savings. Secondly, lower operation temperatures can contribute to the long-term stability of the catalyst, as any deactivation due to higher temperatures can be mitigated. Enhanced catalyst lifetimes may therefore be achieved. Furthermore, reduced amounts of unwanted by-products simplify the process work-up section. Lastly, reduction of unwanted by-products leads to the formation of higher quantity of valuable main product, thus improving profitability.

In an embodiment, the total amount of copper (calculated as CuO) and silica (SiO₂) in the catalyst is in the range of from 81 to 100 % by weight of the catalyst.

As discussed further hereinbelow, the catalyst may be prepared by a precipitation method wherein the copper is added to the catalyst by precipitating a solution comprising copper. However, notwithstanding the specific copper solution used in the preparation of the catalyst, the skilled person will understand that the copper present in the resulting active catalyst after reduction will be present as one or more of copper metal (Cu), copper (I) oxide (Cu₂O) and/or copper (II) oxide (CuO).

Other components that may be present in the catalyst composition may include, for example, zirconia and titania and, optional promoters.

Percentages "by weight of the catalyst" herein refer to percentages of the weight of the total dry catalyst. Suitably, the weight of the total dry catalyst may be measured following drying for at least four hours at 120 to 150 °C.

Percentages of metal or metal oxide in the catalyst may be determined by XRF as is known in the art. The metal content of catalysts may also be inferred or controlled via their synthesis.

In a preferred embodiment, the catalyst comprises copper (calculated as CuO) in an amount in the range of from 71 to 85 % by weight, more preferably in the range of from 71 to 80 % by weight and most preferably in the range of from 72 to 75 % by weight, of the catalyst.

In a preferred embodiment, in addition to copper (calculated as copper oxide), the balance of the catalyst comprises silica.

Generally, the catalyst comprises silica (SiO₂) in an amount in the range of from 10 to 29 % by weight of the catalyst. Preferably, the catalyst comprises silica (SiO₂) in an amount in the range of from 15 to 29 % by weight, more preferably from 20 to 29 % by weight and most preferably from 25 to 28 % by weight, of the catalyst.

However, in some embodiments of the present invention, the catalyst may further comprise one or more alkali and/or alkaline earth metal compounds, which may have a promoting effect. The preferred alkali metal is sodium and the preferred alkaline earth metal is calcium. Suitably, the alkali and/or alkaline earth metal compound(s) may be present in a total amount of up to 7.0 % by weight, preferably up to 5.0 % by weight, more preferably up to 3.0 % by weight, and most preferably up to 1.0 % by weight, of the catalyst.

In a preferred embodiment, the catalyst has a BET surface area greater than 50 m²/g, more preferably greater than 80 m²/g, as measured with nitrogen adsorption according to ASTM D4567.

In a preferred embodiment, the catalyst has a pore volume greater than 0.3 cm³/g, more preferably greater than 0.4 cm³/g, as measured by mercury intrusion porosimetry according to ASTM D4284.

In a preferred embodiment, when the catalyst is in tablet form, the catalyst has a crush strength of at least 200 N, more preferably in the range of from 300 N to 500 N, as measured according to ASTM D4179.

In another preferred embodiment, when the catalyst is an extrudate, the catalyst has a crush strength of at least 3 N/mm, more preferably at least 5 N/mm and most preferably at least 10 N/mm, as measured according to ASTM D6175.

The catalyst may in principle be prepared by any suitable technique known in the art. Advantageously, the catalyst may be prepared by precipitation.

The components of the catalyst, are to be selected in an overall amount not to exceed 100 wt. %.

In the first step of the catalyst preparation, a solution comprising copper salts, preferably an aqueous solution, is prepared.

In case the catalyst is to contain one or more additional metals, the solution should also comprise a salt of the additional metal(s).

The copper salt may be a nitrate, sulfate, chloride or organic acid salt of copper. Preferably, the copper salt is copper nitrate or an organic acid salt of copper, most preferably copper nitrate. A suitable organic acid salt of copper is copper acetate.

If used, the salt of the additional metal(s) may be a nitrate, sulfate, chloride or organic acid salt of the additional metal. Preferably, the salt of the additional metal(s) is a nitrate of the additional metal or an organic acid salt of the additional metal, most preferably a nitrate of the additional metal. A suitable organic acid salt of the additional metal is an acetate of the additional metal.

The solution comprising the metal salts thus obtained should then be combined with a basic solution in order to effect precipitation of the solution comprising the metal salts, resulting in a precipitate (in a slurry or dispersion) comprising copper.

Any basic solution effecting such precipitation may be used. Preferably, the basic solution is an aqueous solution. Further, preferably, the basic solution comprises one or more bases selected from the group consisting of sodium hydroxide, sodium carbonate, sodium hydrogen carbonate, potassium hydroxide, potassium carbonate, potassium hydrogen carbonate, ammonium carbonate and ammonium hydrogen carbonate. Preferably, sodium carbonate is used as the base in the basic solution. In addition to the base, the basic solution may comprise one or more of the above-mentioned metal salts.

The amount of base in said basic solution should be sufficient to allow for precipitation of all of the metal salts. This may be achieved by using such amount of base that a pH of 8 or greater, preferably a pH of 8.5 or greater, is achieved in the dispersion. Preferably, in the precipitation step, the combined solution comprising the base and the metal salts is heated, for example at 50 to 95 °C, preferably 70 to 95 °C.

The solid (precipitate) in the dispersion or slurry resulting from combining the solution comprising the metal salts with a basic solution, can be recovered therefrom by filtration. The separated solid may be washed with water, preferably deionized, alkali metal free water, for example in order to remove substantially all alkali metal ions that originate from the basic solution. Possibly after drying the separated solid, for example at a temperature in the range of from 80 to 160 °C, suitably 100 to 140 °C, it may further be calcined in the presence of air, at a temperature in the range of from 200 to 800 °C, preferably 250 to 700 °C, more preferably 300 to 600 °C, most preferably 350 to 500 °C

In addition, an auxiliary for making shaped catalyst bodies comprising tablets and extrudates may be mixed with the catalyst. An example of such auxiliary is a binder, for example graphite or a silica precursor. If such auxiliary is used, the amount used is preferably in the range of from 0.1 to 20 wt.%, more preferably in the range of from 0.1 to 10 wt.%, and most preferably in the range of from 1 to 5 wt.%, based on the total weight of the catalyst.

In a preferred embodiment, the catalyst is obtained by a method comprising: preparing an acidic solution comprising a source of copper, a source of silica, and optionally one or more alkali metals; adding the acidic solution to an alkaline solution to form a precipitate; filtering and drying the precipitate; calcining the dried precipitate; and optionally shaping the calcined precipitate, e.g. into pellets, to provide the catalyst. In an embodiment, the catalyst is calcined at a temperature in the range of from 300 to 500 °C.

The catalyst may be a passivated catalyst for subsequent activation by reduction as is known in the art. Alternatively, the catalyst may be in reduced form and optionally stabilized.

Particular examples of suitable catalysts are copper/silica catalysts available from CRI Catalyst Leuna GmbH such as "KL 2012" catalyst.

The feed of phenyl alcohol contacted with the heterogeneous catalyst may comprise one or more phenyl alcohols.

Suitably, the structure of the alcohol may comprise a single hydroxyl group to be eliminated during hydrogenolysis. In the hydrogenolysis eliminated hydroxyl groups are replaced by hydrogen.

A phenyl alcohol is an alcohol comprising a phenyl ring. Suitably, the phenyl alcohol is an alkyl phenyl alcohol, i.e. an alcohol comprising or consisting of a phenyl ring and a hydroxyl-bearing alkyl group. Preferably said alkyl group may contain 2 to 4 carbon atoms.

In an embodiment, the alcohol is a secondary alcohol or a tertiary alcohol.

In an embodiment, the phenyl alcohol is a phenyl carbinol. In an embodiment, the phenyl alcohol is a methyl-branched phenyl carbinol. As set out above, such phenyl carbinols are of particular use in the production of propylene oxide. In an embodiment, the phenyl alcohol is methylphenylcarbinol (1-phenylethanol) or preferably dimethylphenylcarbinol (DMPC) (2-phenylpropan-2-ol) (cumyl alcohol).

The phenyl alcohol may preferably comprise an unsubstituted phenyl ring, by which is meant a phenyl ring unsubstituted save for the hydroxyl-comprising substituent forming the alcohol. However, the phenyl ring may also be substituted. An example of a substituted phenyl alcohol is cumic alcohol (4-isopropylbenzyl alcohol). In an embodiment, the phenyl ring is either unsubstituted or comprises one or more substituents selected from alkyl groups having 1 to 6 carbon atoms, in particular 1 to 3 carbon atoms. Preferably, no other substituents are present.

The method comprises contacting a feed comprising the phenyl alcohol and hydrogenating agent with the heterogeneous catalyst under hydrogenolysis conditions. Conveniently, the hydrogenating agent may be hydrogen. The catalyst may conveniently be supported in a fixed bed.

In an embodiment, the feed is contacted with the catalyst to achieve a conversion of at least 95 % by weight, preferably at least 98 % by weight, more preferably at least 99 % by weight, even more preferably at least 99.5 % by weight of the phenyl alcohol (e.g. DMPC). Advantageously, the selectivity towards the hydrogenolysis product, i.e. corresponding compound with eliminated hydroxyl group (e.g. cumene), may be at least 99 % wt./wt.

The method of the present invention is carried out at a temperature of less than 140 °C. In a preferred embodiment, the method comprises contacting the phenyl alcohol with the heterogeneous catalyst at a temperature in the range of from 100 to less than 140 °C, preferably in the range of from 110 to 138 °C, more preferably in the range of from 120 to 138 °C.

The method may be carried out at any suitable pressure, e.g. a pressure in the range of from 100 to 10,000 kPa. However, as aforesaid, the specific heterogeneous catalyst comprising copper and silica described herein has been found to offer particularly high levels of activity. Accordingly, the method may advantageously be carried out at relatively low pressures whilst maintaining high levels of conversion.

In an embodiment, the method comprises contacting the phenyl alcohol with the heterogeneous catalyst at a pressure in the range of from 300 to 5000 kPa, preferably in the range of from 500 to 3000 kPa and more preferably in the range of from 1000 to 2000 kPa.

The composition of the feed may be varied in any suitable manner. In an embodiment, the molar ratio (mol/mol) of hydrogen/phenyl alcohol is in the range of from 2 to 20, preferably in the range of from 4 to 10, more preferably in the range of from 6 to 8. Where a hydrogenating agent other than hydrogen is used, molar ratios may be calculated on the basis of equivalence to hydrogen.

The feed may comprise one or more other components or may consist of the hydrogenation agent and the phenyl alcohol(s). In an embodiment, the feed comprises an amount of a solvent, such as for example a hydrogenolysis product of the phenyl alcohol(s). In an embodiment, the phenyl alcohol(s), and optionally solvent, make up at least 95 % wt., e.g. at least 98 % wt., of an organic part of the feed, i.e. a part excluding the hydrogenation agent. Any remainder of the organic part of the feed may comprise trace components of other compounds, as is known in the art, for example trace components resulting from an epoxidation reaction. In one embodiment, a solvent is present in the feed and the phenyl alcohol(s) makes up in the range of from 10 to 60 % wt./wt. of the organic part of the feed, in particular in the range of from 20 to 40 % wt./wt.

The reaction may be conducted in the liquid and/or vapour phase. In an embodiment, the catalyst is retained in a catalyst bed. In another embodiment, the catalyst is in the form of a catalyst slurry.

The feed rate of the feed may be adjusted in any suitable manner. In an embodiment, the liquid hourly space velocity (LHSV) of the feed is maintained in the range of from 0.1 to 2 litres per kilogram of heterogeneous catalyst per hour (1/kg cat/h), preferably in the range of from 0.5 to 1.5 1/kg cat/h, most preferably in the range of from 0.7 to 1.2 1/kg cat/h.

In one particular preferred embodiment of the present invention, there is provided a method for the hydrogenolysis of DMPC to cumene, the method comprising contacting a feed comprising DMPC and hydrogen with a heterogeneous catalyst at a temperature in the range of from 100 to less than 140 °C, the catalyst comprising: copper in an amount in the range of from 71 to 90 % by weight of the catalyst and silica (SiO₂) in an amount in the range of from 10 to 29 % by weight of the catalyst.

As aforesaid, the present invention finds particular benefit in the production of alkylene oxides, such as propylene oxide. Accordingly, from a second aspect, the invention resides in a process for the preparation of an alkylene oxide, which process comprises: contacting a phenyl hydroperoxide and an alkene with an epoxidation catalyst in an epoxidation step and withdrawing a product stream comprising an alkylene oxide and a phenyl alcohol; separating alkylene oxide; and converting the phenyl alcohol by hydrogenolysis to eliminate a hydroxyl group therefrom in accordance with the method of the first aspect of the invention. Optionally, the process may further comprise converting the hydrogenolysis product to a phenyl hydroperoxide for recycling to the epoxidation step. Suitable epoxidation catalysts, for example titanium-silica catalysts, and conditions are known in the art, e.g. from EP 0345856 A1 or EP 1433770 A1 and EP 1437350 A1.

Preferably, the alkylene oxide may be propylene oxide, with the alkene being propylene.

As aforesaid, alkyl phenyl hydroperoxides are preferred, particularly in the context of propylene oxide production. The phenyl alcohol is then a corresponding alkyl phenyl alcohol.

In an embodiment, the phenyl hydroperoxide is cumene hydroperoxide (2-hydroperoxypropan-2-ylbenzene) and the phenyl alcohol is DMPC (2-phenylpropan-2-ol) (cumyl alcohol).

In an embodiment, the phenyl hydroperoxide is EBHP and the phenyl alcohol is 1-phenylethanol.

Throughout the description and claims of this specification, the words "comprise" and "contain" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Other features of the invention will become apparent from the following examples. Generally speaking the invention extends to any novel one, or any novel combination, of the features disclosed in this specification (including any accompanying claims and drawings). Thus, features, integers, characteristics, compounds, chemical moieties or groups described in conjunction with a particular aspect, embodiment or example of the invention are to be understood to be applicable to any other aspect, embodiment or example described herein unless incompatible therewith. Moreover unless stated otherwise, any feature disclosed herein may be replaced by an alternative feature serving the same or a similar purpose.

Where upper and lower limits are quoted for a property then a range of values defined by a combination of any of the upper limits with any of the lower limits may also be implied.

In this specification, references to properties are - unless stated otherwise - to properties measured under ambient conditions, i.e. at atmospheric pressure and at a temperature of about 20°C.

The present invention will now be further described with reference to the following non-limiting examples.

### Example 1

A range of heterogeneous catalysts were screened to determine their activity in the hydrogenolysis of DMPC (2-phenylpropan-2-ol) (cumyl alcohol). The experiments were carried out in a tubular reactor containing the catalysts in continuous mode of operation.

Example Catalyst 1 was "KL2012" catalyst commercially available from CRI Catalyst Leuna GmbH. This catalyst comprised 72 wt. % copper oxide (CuO), 3 wt. % of alkali and alkaline earth metal compounds as promoters and silica as balance.

The catalyst had a BET surface area in excess of 120 m²/g, measured by nitrogen adsorption according to ASTM D4567 and a pore volume in excess of about 0.32 g/cm³, measured by mercury intrusion porosimetry according to ASTM D4284.

The catalyst was reduced in a hydrogen-containing stream at a temperature of up to 220 °C prior to use.

Comparative Catalyst A was "G22-2" catalyst obtained from Süd Chemie Corp, which is a copper-chromium catalyst with about 39 wt. % copper and about 24 wt. % chromium, with barium as a promoter.

Comparative Catalyst B was obtained from CRI Catalyst Leuna GmbH. This catalyst comprised about 0.5 % by weight of the catalyst palladium and a balance of alumina. It had a BET surface area of about 115 m²/g.

For each catalyst, following any required activation/reduction, a feed of hydrogen and 2-phenylpropan-2-ol (DMPC) in a molar ratio of about 8 mol/mol was contacted with the catalyst at a liquid hourly space velocity of about 1 1/kg cat/h and a pressure of 1400 kPa (14 barg).

A conversion of DMPC in excess of 99.5 wt. % (i.e. less than 0.5 wt. % DMPC remaining in the product) was targeted for each catalyst and the necessary reaction temperature recorded. The results are shown in Table 1.

**Table 1**

| **Catalyst** | **Reaction Temp (°C)** | **DMPC conversion (wt. %)** |
|---|---|---|
| Example Catalyst 1 | 128 | 94.55 |
| Example Catalyst 1 | 137 | 99.73 |
| Comparative Catalyst A | 135 | 97.34 |
| Comparative Catalyst A | 155 | 99.97 |
| Comparative Catalyst B | 143 | 81.30 |
| Comparative Catalyst B | 175 | 99.84 |

The calculated temperatures required for achieving the target conversion of 99.5 wt. % (derived from the data in Table 1) for the three catalysts are depicted in Table 2.

**Table 2**

| **Catalyst** | **Required temp (°C) (for 99.5 wt. % DMPC conversion)** |
|---|---|
| Example Catalyst 1 | 136.6 |
| Comparative Catalyst A | 151.4 |
| Comparative Catalyst B | 174.4 |

It is apparent that it was surprisingly [possible for the copper -silica catalyst indicated as Example Catalyst 1 to be operated at a temperature about 15°C lower than the copper-chromium Comparative Catalyst A and about 40°C lower than palladium Comparative Catalyst B for comparable conversion.

Example Catalyst 1 thus showed significantly higher activity.

### Example 2

The selectivity of Example Catalyst 1 and Comparative Catalyst A in the hydrogenolysis of DMPC was compared.

For each catalyst, a fixed bed was prepared and, following activation/reduction, a liquid feed comprising of 35 wt. % DMPC and 63 wt. % cumene, spiked with impurities to the extent of 1 wt. % methyl phenyl ketone (acetophenone), 0.3 wt. % phenol and 0.015 wt. % benzoic acid and balance of other organic trace components (no ring hydrogenated cumene or cumene dimer), and hydrogen in a molar ratio of 8 mol/mol to DMPC, was contacted with the catalyst at a liquid hourly space velocity of about 1/kg cat/h and a pressure of 1400 kPa (14 barg) over a period of four hours.

To obtain a suitably high conversion for both catalysts, the same reaction temperature of 155 °C was set for both Example Catalyst 1 and Comparative Catalyst A.

The product obtained from contacting the aforementioned liquid feed with Example Catalyst 1 was found to contain 98.39 wt. % cumene, which represents a selectivity to the primary hydrogenolysis product in excess of 99% wt./wt. The amount of ring hydrogenated cumene in the product was 0.14 wt. %, and 0.02 wt. % cumene dimer was detectable.

The product obtained from contacting with Comparative Catalyst A was found to contain 98.35 wt. % cumene, which represents a catalyst selectivity in excess of 99% wt./wt. to the primary hydrogenolysis product, but lower than the selectivity of Example Catalyst 1. The amount of ring hydrogenated cumene was similar at 0.14 w%, but cumene dimer was present in an amount of 0.36 w%.

Considering that dimers indicate a direct loss of valuable final products, it can be concluded that the selectivity is not as good for Comparative Catalyst A as for Example Catalyst 1.

In conclusion, Example Catalyst 1 offered excellent selectivity and produced lower amounts of key by-products than Comparative Catalyst A.

## Claims

1. A method for the hydrogenolysis of a phenyl alcohol to eliminate a hydroxyl group therefrom, the method comprising contacting a feed comprising the phenyl alcohol and a hydrogenating agent with a heterogeneous catalyst comprising copper and silica at a reaction temperature of less than 140 °C, wherein the catalyst comprises copper (calculated as CuO) in an amount in the range of from 71 to 90 % by weight of the catalyst.

2. The method of claim 1, wherein the catalyst comprises silica (SiO₂) in an amount in the range of from 10 to 29 % by weight of the catalyst.

3. The method of claim 1 or 2, wherein the catalyst further comprises one or more alkali and/or alkaline earth metal compounds in a total amount of up to 7.0 % by weight of the catalyst.

4. The method of any preceding claim, wherein the phenyl alcohol is a methyl-branched phenyl carbinol.

5. The method of any preceding claim, wherein the phenyl alcohol is DMPC (2-phenylpropan-2-ol).

6. The method of any preceding claim, wherein the feed is contacted with the catalyst under hydrogenolysis conditions to achieve a conversion of at least 99 % by weight of the phenyl alcohol to its corresponding hydrogenolysis product.

7. The method of any preceding claim, wherein the phenyl alcohol is contacted with the heterogeneous catalyst at a temperature in the range of from 100 to less than 140°C.

8. The method of claim 7, wherein the temperature is in the range of from 110 to 138 °C.

9. The method of claim 8, wherein the temperature is in the range of from 120 to 138°C.

10. A process for the preparation of an alkylene oxide, which process comprises:
contacting a phenyl hydroperoxide and an alkene with an epoxidation catalyst in an epoxidation step and withdrawing a product stream comprising an alkylene oxide and a phenyl alcohol;
separating alkylene oxide;
converting the phenyl alcohol by hydrogenolysis to eliminate a hydroxyl group therefrom in accordance with the method of any preceding claim; and, optionally,
converting the hydrogenolysis product to a phenyl hydroperoxide for recycling to the epoxidation step.

11. The process of claim 10, wherein the alkylene oxide is propylene oxide and the alkene is propylene.

12. The process of claim 10 or claim 11, wherein the phenyl hydroperoxide is cumene hydroperoxide (2-hydroperoxypropan-2-ylbenzene) and the phenyl alcohol is DMPC (2-phenylpropan-2-ol).
